# EUROPEAN PATENT APPLICATION

(11) **EP 4 317 454 A1**
(43) Date of publication of application: **07.02.2024**
(21) Application number: 22780163.6
(22) Date of filing: 17.03.2022
(51) Int. Cl.: C12Q 1/6806, C12M 1/00, C12N 15/09, C12N 15/10, C12Q 1/6837

(54) **METHOD FOR DETECTING MICRORNA IN BIOLOGICAL SAMPLE, COMPOSITION OF HYDROCHLORIDE OF NEUTRAL AMINO ACID, AND CONTAINER**

(30) Priority: 29.03.2021 JP 2021054811; 27.07.2021 JP 2021122220
(71) Applicant: Toray Industries, Inc., Tokyo 103-8666 (JP)
(72) Inventor: KOSHI, Yoichiro, Kamakura-shi, Kanagawa 248-8555 (JP); WAKAO, Osamu, Kamakura-shi, Kanagawa 248-8555 (JP)
(74) Representative: Mewburn Ellis LLP
(86) International application number: PCT/JP2022/012206
(87) International publication number: WO 2022/209943

(57) **Abstract**

The microRNA detection level decreases when a microRNA-containing biological sample is preserved after being collected. Further, a relatively long period of time is required for dissolving a hydrochloride of a neutral amino acid, which is in the form of crystals, in water. Since a hydrochloride of a neutral amino acid is an acidic substance, and causes skin irritation and mucosal irritation, the handling of the hydrochloride requires safety precautions. The present invention provides a method of detecting a microRNA in a biological sample, the method including the steps of: mixing an acid with the biological sample, preserving the resulting mixture for a predetermined period of time, collecting the microRNA in the biological sample, and detecting the microRNA. Further, the present invention provides a homogeneous semi-solid composition composed of a hydrochloride of a neutral amino acid, water and an alcohol.

## Description

### Technical Field

The present invention relates to a method of detecting a microRNA in a biological sample. Further, the present invention relates to a composition containing a hydrochloride of a neutral amino acid.

### Background Art

Based on recent developments in gene analysis technology, attempts have been done to detect microRNAs in biological samples, and to utilize them for the diagnosis or treatment of diseases. The detection of a microRNA related to a disease in a biological sample, such as blood, possibly allows for the detection of the disease at an early stage.

On the other hand, microRNAs are contained in extremely small amounts in biological samples, such as blood, as compared with general RNAs on which various studies have been done so far, and are easily degraded by RNA degrading enzymes contained in biological samples. Since microRNAs used as markers in the diagnosis of cancer, in particular, are present extracellularly in serum or a body fluid, such microRNAs are more susceptible to RNA degrading enzymes as compared with general RNAs present intracellularly.

Therefore, biological samples to be used for the detection of microRNAs are usually used immediately after the collection of the biological samples. Alternatively, biological samples that have been collected need to be preserved in a state where the degradation of microRNAs is inhibited by a method such as freezing, and then used for the detection. As described above, the handling of biological samples requires extra precautions in the case of detecting microRNAs, as compared with the case of other RNAs.

In industrial settings, clinical sites at which biological samples are collected and inspection agencies at which the detection of microRNAs is performed are apart from each other, in many cases, and the biological samples need to be preserved for a long period of time, extending from several hours to several days, during which the biological samples are transported from the clinical sites to the inspection agencies.

Biological samples to be used for the detection of microRNAs are usually preserved frozen at an extremely low temperature of -70°C or lower. However, freezing facilities and transportation materials for preserving the biological samples at an extremely low temperature are expensive, and methods are needed that allow for preserving the biological samples to be used for the detection of microRNAs for a long period of time, by a method other than freezing.

To inhibit the degradation of RNAs in a biological sample, a method in which a guanidium salt is added to the biological sample to modify RNA degrading enzymes, or a method in which a synthesized small molecule inhibitor is mixed to the biological sample, is known, in addition to freezing. However, as a more effective and easy method to inhibit the degradation of RNAs, a method is known in which the pH of a biological sample is adjusted to a pH range within which the activity of RNA degrading enzymes is decreased.

Patent Literature 1 discloses a method in which: the pH of a biological sample is adjusted to 4.0 or less at which the activity of RNA degrading enzymes is decreased, to inhibit the degradation of RNAs; then RNA degrading enzymes are inactivated; and thereafter, the pH of the biological sample is adjusted to higher than 6.0, and utilize RNAs.

Hydrochlorides of neutral amino acids including glycine hydrochloride are compounds mainly used in the field of biochemistry. Since hydrochlorides of neutral amino acids have a characteristic to reversibly dissociate bonds between antibodies and antigens, in particular, such compounds are used, for example, in antibody elution in the affinity purification of antibodies, the washing of primary antibodies in multiple immunostaining, and the examination of autoantibodies bound to erythrocytes.

Such compounds are also used in the fields other than biochemistry, and used, for example, in the solubilization of poorly soluble polysaccharides, since they have a characteristic to solubilize substances poorly soluble in water (Non-patent Literature 1).

### Citation List

### Patent Literature

Patent Literature 1: JP 8-508637 A

### Non-patent Literature

Non-Patent Literature 1: Journal of Applied Polymer Science, 123, 3772-3780 (2011).

### Summary of Invention

### Technical Problem

In order to perform the diagnosis or treatment of a disease using a microRNA, a method is needed that allows for detecting the microRNA which is present in an extremely small amount and which is easily degradable, without causing a decrease in the detection level of the microRNA. An object of the present invention is to satisfy such needs, and to provide a method of detecting a microRNA in a microRNA-containing biological sample, without causing a decrease in the detection level of the microRNA, even in cases where the biological sample has been preserved for a long period of time.

Glycine hydrochloride is a solid which is in the form of crystals at normal temperature. Therefore, glycine hydrochloride is often used in a state of an aqueous solution, when used in the applications as described above. An aqueous solution of glycine hydrochloride is prepared by dissolving the solid (crystals) of glycine hydrochloride in water (such as distilled water). However, since a relatively long period of time is required in order to completely dissolve glycine hydrochloride, a sufficient stirring is needed. Further, since glycine hydrochloride is an acidic substance and causes skin irritation and mucosal irritation, the handling of glycine hydrochloride requires safety precautions, particularly during transportation and the like, for example, the necessity to prevent scattering in a solid state (powder form) or splashing in the state of an aqueous solution. Another object of the present invention is to provide a composition which can be used easily and safely in the above-described method of detecting a microRNA.

### Solution to Problem

In order to examine whether or not it is possible to detect a microRNA in a biological sample which has been preserved for a long period of time by a method other than freezing, at a level equal to that before the preservation, the present inventors first compared the detection levels of the microRNA in a biological sample before and after preserving the sample for a long period of time without freezing.

In cases where the diagnosis or treatment of a disease is intended, the value obtained by calculating the quantified value of a specific microRNA or a combination of such quantified values has been used for the diagnosis or treatment. In such a case, the diagnosis or treatment of the disease is significantly compromised when the detection level of each microRNA is decreased, for example, to less than 70%; sometimes, even a decrease to less than 90% could lead to such a result.

Since the combination of microRNAs to be quantified varies depending on the target disease or treatment, it is not realistic to find conditions under which the fluorescence intensities of all microRNAs do not decrease. Therefore, a method is known in which the change in the detection level of all microRNAs arranged in a microarray is estimated by the sum of the fluorescence intensities of all microRNAs detected by the microarray, instead of measuring the change in the detection level of each microRNA. The present inventors performed examinations, using the sum of the fluorescence intensities of all microRNAs on a microarray as the detection level of the microRNAs, and setting the detection level corresponding to 70% with respect to that before the preservation of a biological sample, as the threshold which can be used in the diagnosis or treatment of a disease.

As shown in Comparative Example 4 to be described later, when the serum after the adjustment was preserved at 23°C for 48 hours, and then the detection of microRNAs was performed, the detection level of the microRNAs decreased to 28.9% of the detection level (Comparative Example 5) before the preservation.
That is, it has been revealed that, in a biological sample which has been preserved for a long period of time without being frozen, the detection level of the microRNAs is drastically decreased as a result of degradation by RNA degrading enzymes, as compared with that before the preservation.

As a result of intensive studies to solve the above-mentioned problem, the present inventors have found out that, by preserving a biological sample for a predetermined period of time after mixing an acid with the biological sample, and then collecting a microRNA(s), it is possible to detect the microRNA(s) at a level equal to or higher than that before the preservation.

Further, as a result of intensive studies to find a composition of a hydrochloride of a neutral amino acid, which has a higher solubility, in order to solve the problems of the dissolution rate and safety of glycine hydrochloride as described above, the inventors have discovered a homogeneous semi-solid composition composed of a hydrochloride of a neutral amino acid, water and an alcohol, and found out that this composition is a composition whose solubility in water is improved as compared with that of a conventional solid (crystals) of hydrochloride of a neutral amino acid.

Specifically, the present invention provides the following (1) to (18).
(1) A method of detecting a microRNA in a biological sample, the detection method including the steps of
   (A) mixing an acid with the biological sample to obtain a mixed liquid of the biological sample;
   (B) preserving the mixed liquid of the biological sample obtained in the step (A);
   (D) collecting the microRNA from the mixed liquid of the biological sample obtained in the step (B); and
   (E) detecting the microRNA collected in the step (D).
(2) The detection method of (1), wherein the mixed liquid of the biological sample obtained in the step (A) has a pH of from 2.0 to 4.0.
(3) The detection method of (1) or (2), including, before the step (D), the step (C) of mixing a base with the mixed liquid of the biological sample obtained in the step (B), to adjust the pH of the mixed liquid within the range of from 6.0 to 9.0.
(4) The detection method of (3), wherein the pH of the mixed liquid is adjusted within the range of from 7.5 to 9.0 in the step (C).
(5) The detection method of any one of (1) to (4), wherein the biological sample is blood, serum or plasma.
(6) The detection method of any one of (1) to (5), wherein the acid used in the step (A) is glycine hydrochloride, alanine hydrochloride, citric acid, hydrochloric acid, sulfuric acid, acetic acid, lactic acid or oxalic acid.
(7) The detection method of any one of (1) to (5), wherein the acid used in the step (A) is a homogeneous composition which is composed of a hydrochloride of a neutral amino acid, water and an alcohol, and which is in a semi-solid state at 23°C.
(8) The detection method of any one of (1) to (7), wherein the mixed liquid of the biological sample is preserved for 96 hours or less in the step (B).
(9) The detection method of any one of (1) to (8), wherein the mixed liquid of the biological sample is preserved at a temperature of from 1 to 30°C in the step (B).
(10) The detection method of (3) or (4), wherein the base used in the step (C) is tris(hydroxymethyl)aminomethane, N-[tris(hydroxymethyl)methyl]glycine, triethanolamine, sodium hydroxide, potassium hydroxide, calcium hydroxide, N,N-bis(2-hydroxyethyl)glycine, N-(2-hydroxyethyl)piperazine-N'-2-ethanesulfonic acid or 2-hydroxy-3-[tris(hydroxymethyl)methylamino]-1-propanesulfonic acid.
(11) The detection method of any one of (1) to (10), wherein the microRNA is detected by the microarray analysis in the step (E).
(12) The detection method of any one of (1) to (11), wherein the microRNA is a microRNA to be used as a disease marker.
(13) The detection method of (12), wherein the disease is cancer or dementia.
(14) A composition which is used as the acid in the step (A) in the detection method of (1), wherein the composition is a homogeneous composition which is composed of a hydrochloride of a neutral amino acid, water and an alcohol, and which is in a semi-solid state at 23°C.
(15) The composition of (14), wherein the weight ratio of the hydrochloride of a neutral amino acid in the composition is 65 wt% or more and 70 wt% or less, and the weight ratio of the alcohol therein is 2 wt% or more and 6 wt% or less.
(16) The composition of (14) or (15), wherein the hydrochloride of a neutral amino acid is glycine hydrochloride or alanine hydrochloride.
(17) The composition of any one of (14) to (16), wherein the alcohol is methanol or ethanol.
(18) A container for preserving a biological sample, wherein the composition according to any one of (14) to (17) is encapsulated in the container.

### Advantageous Effects of Invention

The present invention enables to detect a microRNA in a microRNA-containing biological sample, without causing a decrease in the detection level thereof, even in cases where the biological sample has been preserved for a long period of time after being collected, for example, under non-freezing conditions.

Further, the composition according to the present invention can be quickly dissolved in water, as compared with the solid of a hydrochloride of a neutral amino acid. Since the present composition is a homogeneous semi-solid composition, there is no need for safety concerns regarding the scattering of the composition in a solid state (powder form) or splashing in the state of an aqueous solution, which has been a problem of the solid or the aqueous solution of a hydrochloride of a neutral amino acid, and the composition can be handled easily particularly during transportation and the like.

### Description of Embodiments

The present invention provides a method of detecting a microRNA in a biological sample, the detection method including the steps of
(A) mixing an acid with the biological sample to obtain a mixed liquid of the biological sample;
(B) preserving the mixed liquid of the biological sample obtained in the step (A) for a predetermined period of time;
(D) collecting the microRNA from the mixed liquid of the biological sample obtained in the step (B); and
(E) detecting the microRNA collected in the step (D).
The present invention will be described below for each of the above-described steps.

The step (A) of the present invention is the step of mixing an acid with the biological sample to obtain a mixed liquid of the biological sample. By adjusting the pH of the biological sample preferably within the range of from 2.0 to 4.0, more preferably within the range of from 2.5 to 4.0, the activity of RNA degrading enzymes in the biological sample is decreased, and the degradation of the microRNA in the biological sample is reduced. The pH of the biological sample is preferably adjusted to 2.0 or more, because the cleavage of a phosphodiester bond of the microRNA due to acid hydrolysis can be prevented. Further, the pH of the biological sample is preferably adjusted to 4.0 or less, because the enzymatic activity of RNA degrading enzymes contained in the biological sample can be sufficiently reduced.

The "biological sample" to be used in the present invention refers to a sample which can be obtained from a living body including microRNAs. Specific examples of the biological sample include serum, plasma, whole blood, bone marrow fluid, lymph fluid, saliva, bile, pancreatic juice, ascites, body fluids and secretions. In addition, the biological sample may be a tissue obtained from a living body, or a culture of cultured cells or the like.

The biological sample is not particularly limited as long as the sample is collected from an organism, but is preferably derived from a human. In particular, the biological sample to be used in the present invention is preferably a human-derived blood, serum or plasma, and more preferably a human-derived serum or plasma.

The method for collecting a biological sample is well-known in the art. For example, blood can be obtained by suction using a blood collection needle, by a person who collects blood such as a skilled medical professional, and thereafter, serum or plasma can be obtained by centrifugation of the blood.

The acid to be mixed with the biological sample is an acid which can be mixed with the biological sample in the step A. Examples of the acid to be used in the present invention include glycine hydrochloride, alanine hydrochloride, citric acid, hydrochloric acid, sulfuric acid, acetic acid, lactic acid and oxalic acid. In particular, glycine hydrochloride or citric acid is preferred. Further, a homogeneous composition which is composed of a hydrochloride of a neutral amino acid, water and an alcohol, and which is in a semi-solid state at 23°C, is also preferred.

The method of mixing the acid with the biological sample is not particularly limited. For example, the biological sample may be introduced into a container into which the acid is introduced in advance, and a micropipette or the like may be used to perform mixing by pipetting. Alternatively, for example, the biological sample may be introduced into a sealable container into which the acid is introduced in advance, and after sealing the container, mixing may be performed by shaking the container using a shaker such as a vortex mixer, or by turning the container upside down. In particular, the method of mixing by turning the container upside down allows for a quick mixing, even in an environment without special equipment, such as, for example, in a vehicle for medical checkup, etc., used for regular medical checkup or the like.

The step (B) of the present invention is the step of preserving the mixed liquid of the biological sample for a predetermined period of time. The "predetermined period of time" in the step (B) refers to a period of time during which the effect of inhibiting the degradation of the microRNA in the biological sample, provided by mixing with the acid, can be obtained, and the time can be selected as appropriate taking into consideration the time until the step (D) is performed.

For example, in the case of suspending the operation temporarily after the step (A), preserving the mixed liquid of the biological sample obtained in the step (A), and then performing the operations of collecting and detecting the microRNA, the thus preserved time can be taken as the predetermined period of time in the step (B). Alternatively, for example, in the case of transporting the mixed liquid of the biological sample obtained in the step (A) after preserving the mixed liquid temporarily, the total of the preservation time and the transportation time can be taken as the predetermined period of time in the step (B).

More specifically, for example, in cases where the location at which a person who collects blood obtains the biological sample and the location at which the operations of collecting and detecting the microRNA are performed are apart from each other, and thus the mixed liquid of the biological sample is placed in a sealable container and preserved at the location at which the sample is obtained, as it is, and then transported, such as in the case of regular medical checkup, etc., the total of the preservation time and the transportation time can be taken as the predetermined period of time in the step (B).

Specifically, the predetermined period of time is preferably one hour or more, as the period of time during which the effect of the present invention can be obtained. In actual operation, in particular, the predetermined period of time is more preferably 24 hours or more, and still more preferably 48 hours or more, taking into consideration the case in which the location at which a person who collects blood obtains the biological sample and the location at which the operations of collecting and detecting the microRNA are performed are apart from each other.

On the other hand, although the period of time during which the effect of the present invention can be obtained can vary depending on the temperature or the type of the biological sample, the predetermined period of time is preferably generally 96 hours or less.

The preservation of the mixed liquid of the biological sample in the step B can usually be performed under temperature conditions in which the collection, transportation and the like of the biological sample are performed, for example, without requiring freezing conditions. Specifically, the preservation is performed at a temperature of from 1 to 30°C, preferably from 15 to 25°C. Particularly, for example, preservation conditions under which a detection level of the microRNA of 70% or more can be achieved in the microarray analysis, in the detection of the microRNA in the step (E), are preferred. More preferred are preservation conditions under which a detection level of the microRNA of 90% or more can be achieved.

In the present steps, it is preferred to add, before the step (D), the step (C) of mixing a base with the mixed liquid of the biological sample, to adjust the pH of the mixed liquid. In the step (C), the pH of the mixed liquid is preferably adjusted within the range of from 6.0 to 9.0 in which the microRNA can be detected at a level equal to that before the preservation. The pH is preferably adjusted within the range of from 7.5 to 9.0 in which the microRNA can be detected at a level equal to or higher than that before the preservation, and more preferably from 7.5 to 8.5. When the pH of the mixed liquid of the biological sample is adjusted to less than 6.0 or to higher than 9.0, there is a risk that the detection level of the microRNA is decreased as compared with that before the preservation.

The base to be mixed in the step (C) is a base capable of adjusting the pH of the mixed liquid of the biological sample within the range of from 6.0 to 9.0, by being mixed with the mixed liquid. Examples of the base include tris(hydroxymethyl)aminomethane (Tris), N-[tris(hydroxymethyl)methyl]glycine (Tricine), triethanolamine, sodium hydroxide, potassium hydroxide, calcium hydroxide, N,N-bis(2-hydroxyethyl)glycine (Bicine), 4-(2-hydroxyethyl)-1-piperazineethanesulfonic acid (HEPES) and 2-hydroxy-3-[tris(hydroxymethyl)methylamino ]-1-propanesulfonic acid (TAPSO). In particular, the base to be used in the present invention is preferably tris(hydroxymethyl)aminomethane.

The method of mixing the base with the mixed liquid of the biological sample is not particularly limited. For example, the base may be introduced into a container into which the mixed liquid of the biological sample is introduced in advance, and a micropipette or the like may be used to perform mixing by pipetting. Alternatively, for example, the base may be introduced into a sealable container into which the mixed liquid of the biological sample is introduced in advance, and after sealing the container, mixing may be performed by shaking the container using a shaker such as a vortex mixer, or by turning the container upside down.

The step (D) of the present invention is the step of extracting and collecting the microRNA. To collect the microRNA, a method well known in the art of nucleic acid collection can be used. Examples include methods such as phenolchloroform extraction, column chromatography, ethanol precipitation and magnetic beads adsorption.

The step (E) of the present invention is the step of detecting the microRNA that has been collected. To detect the microRNA, a method well known in the art of nucleic acid detection can be used. Examples include methods such as microarray analysis method, PCR method, Northern blotting method and sequence analysis method. In particular, the method to be used in the present invention is preferably the microarray analysis method.

The microRNA of the present invention may be, for example, a microRNA to be used as a disease marker. The present invention can provide information for diagnosing a disease, by providing the detection value of the expression level of a microRNA which serves as a disease marker, in a sample. The disease marker of the present invention may be, for example a marker of cancer or dementia.

Further, the present invention relates to a composition composed of a hydrochloride of a neutral amino acid, water and an alcohol.

The composition according to the present invention is characterized in that the composition is in a homogeneous semi-solid state at 23°C, differing from the solid (crystals) of a hydrochloride of a neutral amino acid, an aqueous solution of a hydrochloride of a neutral amino acid, or a suspension in which the solid of a hydrochloride of a neutral amino acid is dispersed.

The homogeneous composition which is composed of a hydrochloride of a neutral amino acid, water and an alcohol and which is in a semi-solid state at 23°C, is preferred as the acid to be used in the step (A) of the present invention, because the composition has advantages that it quickly dissolves in the mixed liquid of the biological sample, and that it is less likely to scatter.

The "semi-solid state" refers to a state in which a shape can be easily deformed when a force is applied thereto, and the shape after the deformation is retained even after the force is removed. A hydrochloride of a neutral amino acid (crystals) is a solid and not easily deformed by a force, and therefore, the above-described definition does not apply thereto. Further, a suspension of a hydrochloride of a neutral amino acid does not retain the shape after the deformation, and therefore, the above-described definition does not apply thereto.

Whether or not the composition is in a semi-solid state is determined by applying a force to the composition to cause deformation, and then examining whether or not the shape after the deformation is retained. Specifically, when the composition is deformed into a cube-like shape with a size of 0.5 cm × 0.5 cm × 1.0 cm height, and if a height of 0.8 cm or more could be retained at 23°C for 10 minutes after the deformation, the composition is determined to be in a semi-solid state.

The composition according to the present invention is characterized in that the composition more quickly dissolves in water at normal temperature, without requiring an operation such as stirring, as compared with the solid of a hydrochloride of a neutral amino acid.

Whether or not the composition quickly dissolves in water can be evaluated by adding the composition to water and observing the manner in which the composition dissolves. When distilled water in a weight 10 times that of the composition is added to a container encapsulating the composition and the container is left to stand for a certain period of time, and if no solid residue is observed in the water thereafter, it is determined that the composition quickly dissolved in water, whereas if solid residues are observed in the water thereafter, it is determined that the composition did not dissolve in water. Specifically, when 1 mL of distilled water is added to a 2 mL PP tube encapsulating 0.1 g of the composition and the tube is left to stand at 23°C for one minute, and if no solid residue is observed in the resulting aqueous solution thereafter, it is determined that the composition quickly dissolved in water, whereas if solid residues are observed in the aqueous solution thereafter, it is determined that the composition did not dissolve in water.

When the solid (crystals) of glycine hydrochloride was evaluated by the method described above, solid residues were observed in the aqueous solution, and thus it was determined that the composition did not dissolve in water (see Comparative Example 11).

The composition according to the present invention is a composition which contains a hydrochloride of a neutral amino acid, water and an alcohol, and which is in a semi-solid state at 23°C.

The composition according to the present invention preferably contains a hydrochloride of a neutral amino acid in a weight ratio of 65 wt% or more and 70 wt% or less, an alcohol in a weight ratio of 2 wt% or more and 6 wt% or less, and water. The weight ratios of the hydrochloride of a neutral amino acid and the alcohol in the composition can be calculated by diluting the composition with water, and then analyzing the diluted composition by gas chromatography. The weight ratio of water in the composition can be calculated by allowing the moisture in the composition to vaporize by heating, using a moisture vaporizer, and then measuring the amount of moisture vaporized using a Karl Fischer moisture meter.

The definition of the hydrochloride of a neutral amino acid to be used in the composition according to the present invention includes a hydrochloride of an amino acid in which the numbers of acidic carboxy groups and basic amino groups contained in the molecule are the same. Examples thereof include, but not limited to, hydrochlorides of amino acids such as glycine, alanine, valine, leucine, isoleucine, serine, threonine, cysteine, methionine, asparagine, glutamine, proline, phenylalanine, tyrosine and tryptophan, which constitute proteins. Of these, glycine hydrochloride or alanine hydrochloride is preferred, from the viewpoints of the cost and availability.

The alcohol to be used in the composition according to the present invention is preferably, for example, an alcohol having from 1 to 8 carbon atoms. Further, the alcohol of the present invention is preferably a monohydric to trihydric alcohol. Specific examples thereof include: monohydric alcohols such as methanol, ethanol, n-propanol, isopropanol, n-butanol, i-butanol, t-butanol, 3-methyl-1-butanol, 1-pentanol, 2-pentanol, n-hexanol, cyclohexanol, 1-heptanol, 1-octanol, 2-octanol, 2-methoxyethanol, allyl alcohol, furfuryl alcohol and phenol; and polyhydric alcohols such as ethylene glycol, propylene glycol, butylene glycol and glycerin. Of these, methanol or ethanol is preferred from the viewpoints of the cost and availability.

In the case of a composition (aqueous solution) consisting of glycine hydrochloride and water, in which the weight ratio of glycine hydrochloride is more than 50 wt%, glycine hydrochloride does not dissolve in water at 23°C, and the composition forms a suspension in which the solid of glycine hydrochloride is dispersed in water. Likewise, in the case of a composition (aqueous solution) consisting of alanine hydrochloride and water, in which the weight ratio of alanine hydrochloride is more than 50 wt%, the alanine hydrochloride does not dissolve in water at 23°C, and the composition forms a suspension in which the solid of alanine hydrochloride is dispersed in water. The composition according to the present invention contains an alcohol in addition to a hydrochloride of a neutral amino acid and water, and as a result, the composition can be obtained as a homogeneous semi-solid composition, not as a suspension.

By dissolving the composition according to the present invention in water, it is possible to easily prepare an aqueous solution of a hydrochloride of a neutral amino acid having a desired concentration. Further, the composition can also be dissolved in an aqueous solvent. The aqueous solvent may be, for example, a buffer solution, a culture medium, a liquid biological sample or the like. The liquid biological sample may be, for example, blood, serum, plasma, urine, saliva, tear fluid or the like.

Examples of the method of dissolving the composition according to the present invention in a solvent include a method in which the composition is added to a solvent, and a method in which a solvent is added to a container in which the composition is encapsulated in advance. The container in which the composition is encapsulated in advance, may be, for example, a plastic tube, a glass tube or the like, but not particularly limited thereto.

As described above, the container in which the composition according to the present invention is encapsulated in advance, may be used as a container for preserving the biological sample. Such a container has advantages that the composition is less likely to scatter when the container is opened, and that the biological sample can be quickly stabilized by acidification when the sample is added to the container.

The amount of the composition according to the present invention to be dissolved in a solvent can be adjusted as appropriate, depending on the target concentration. However, the amount of composition to be dissolved is preferably from 0.01 g to 10.0 g, and more preferably from 0.1 g to 1.0 g, with respect to 100 mL of the solvent.

Since a solution of the composition according to the present invention shows acidity, the composition according to the present invention can be used as a pH regulator, as with a conventional hydrochloride of a neutral amino acid.

In cases where the composition according to the present invention is a composition composed, for example, of glycine hydrochloride, water and methanol or ethanol, the composition can be prepared by:
step (1): the step of mixing glycine hydrochloride, water and methanol or ethanol in a predetermined weight ratio, to obtain a suspension of glycine hydrochloride;
step (2): the step of heating the suspension obtained in the step (1), to dissolve glycine hydrochloride; and
step (3): the step of cooling the solution of glycine hydrochloride obtained in the step (2), to obtain the composition.
The weight ratio of glycine hydrochloride to be mixed in the step (1) is set to 65 wt% or more and 70 wt% or less, the weight ratio of ethanol or methanol is set to 2 wt% or more and 6 wt% or less.

In the case of preparing the composition by the method described above, glycine hydrochloride does not dissolve in the step (1) and the composition is in the form of a suspension in which the solid of glycine hydrochloride is dispersed in water. However, the suspension turns into a homogeneous solution, by being heated in the step (2) to dissolve glycine hydrochloride, and a homogeneous semi-solid composition which is desired can be obtained by cooling in the step (3).

The method of mixing glycine hydrochloride, water and ethanol or methanol in the step (1) is not particularly limited. For example, a method of mixing the components at 23°C for one minute using a vortex mixer can be used.

The temperature at which the suspension is heated in the step (2) can be any temperature which is equal to or lower than the boiling point of a substance having the lowest boiling point in the components, and which is capable of dissolving glycine hydrochloride. For example, the suspension can be heated at 75°C in the case of using ethanol (boiling point: 78.4°C), and at 60°C in the case of using methanol (boiling point: 64.7°C). The heating method is not particularly limited, and a water bath or a block incubator can be used. The heating time is preferably 5 minutes or more and 30 minutes or less. The heating is preferably carried out in a sealed state, in order to prevent the evaporation of water and ethanol or methanol during the heating.

Whether or not glycine hydrochloride dissolved in the step (2) can be determined by visual observation. It is determined that glycine hydrochloride has dissolved if no solid residue is observed in the aqueous solution after heating, and it is determined that glycine hydrochloride has not dissolved if solid residues are observed in the aqueous solution.

The cooling in the step (3) may be performed by a method using a commonly used cooling device. However, the heated aqueous solution may be allowed to cool by standing still, in a container used for heating, as it is, or after being transferred to another container. The aqueous solution is preferably allowed to cool for 10 minutes or more and 60 minutes or less. The temperature after cooling is preferably from 20 to 25°C.

In the case of using alanine hydrochloride, the composition according to the present invention can be prepared by the same steps as in the case of using glycine hydrochloride.

In the case of using a hydrochloride of a neutral amino acid other than glycine hydrochloride and alanine hydrochloride, the composition according to the present invention can be prepared by the same steps.

### Examples

### Example 1 Detection of MicroRNAs from Serum Preserved after Being Mixed with Glycine Hydrochloride - No. 1

### (A) Step of Mixing Acid with Biological Sample

Blood was collected from a healthy subject X, and serum was prepared therefrom. A quantity of 90 µL of a 1 M aqueous solution of glycine hydrochloride (manufactured by FUJIFILM Wako Pure Chemical Industries, Ltd.) was mixed with 300 µL of the serum immediately after the preparation. The pH of the serum was measured using a handy pH meter (manufactured by HORIBA, Ltd.), and as a result, it has been confirmed that the pH decreased from 7.5 to 2.8 by mixing the acid.

### (B) Step of Preserving Mixed Liquid of Biological Sample for Predetermined Period of Time

The serum which had been mixed with the acid in the step (A) was left to stand and preserved at 23°C for 48 hours.

### (C) Step of Mixing Base with Mixed Liquid of Biological Sample

A quantity of 27 µL of a 4 M aqueous solution of tris(hydroxymethyl)aminomethane (manufactured by Nacalai Tesque Inc.) was mixed with the serum preserved in the step (B). Using the handy pH meter, it has been confirmed that the pH of the serum increased from 2.8 to 7.5 by mixing the base.

### (D) Step of Collecting MicroRNAs from Mixed Liquid of Biological Sample

From the serum which had been mixed with the base in the step (C), microRNAs were collected using an RNA extraction reagent included in a 3D-Gene (registered trademark) RNA extraction reagent from liquid sample kit (manufactured by Toray Industries, Inc.), in accordance with the protocol defined by the company.

### (E) Step of Detecting MicroRNAs

The microRNAs collected from the serum in the step (D) were fluorescently labeled, using a 3D-Gene (registered trademark) miRNA Labeling kit (manufactured by Toray Industries, Inc.), and in accordance with the protocol defined by the company. Using a 3D-Gene (registered trademark) Human miRNA Oligo chip (manufactured by Toray Industries, Inc.) containing probes having sequences complementary to 2,565 types of microRNAs, as a DNA microarray chip, hybridization was carried out in accordance with the protocol defined by the company. The DNA microarray chip was scanned using a 3D-Gene (registered trademark) scanner (manufactured by Toray Industries, Inc.), to obtain an image. The fluorescence intensities were digitized using a 3D-Gene (registered trademark) Extraction (manufactured by Toray Industries, Inc.), and the total value of the fluorescence intensities of the microRNAs which had been detected exceeding the detection limit was calculated, and the calculated value was defined as the detection level of the microRNAs. The detection level of the microRNAs is shown in Table 1.

### Examples 2 to 5 Detection of MicroRNAs from Serum Preserved after Being Mixed with Glycine Hydrochloride - No. 2

The same procedure as in Example 1 was performed except that, at the time of mixing the base,
30 µL of the 4 M aqueous solution of tris(hydroxymethyl)aminomethane was mixed to adjust the pH to 7.7, in Example 2,
36 µL of the 4 M aqueous solution of tris(hydroxymethyl)aminomethane was mixed to adjust the pH to 8.0, in Example 3,
60 µL of the 4 M aqueous solution of tris(hydroxymethyl)aminomethane was mixed to adjust the pH to 8.5, in Example 4, and
120 µL of the 4 M aqueous solution of tris(hydroxymethyl)aminomethane was mixed to adjust the pH to 9.0, in Example 5.
The respective detection levels of the microRNAs in the Examples 2 to 5 are shown in Table 1.

### Examples 6 and 7 Detection of MicroRNAs from Serum Preserved after Being Mixed with Glycine Hydrochloride - No. 3

The same procedure as in Example 1 was performed except that, at the time of mixing the base,
21 µL of the 4 M aqueous solution of tris(hydroxymethyl)aminomethane was mixed to adjust the pH to 6.0, in Example 6, and
22.5 µL of the 4 M aqueous solution of tris(hydroxymethyl)aminomethane was mixed to adjust the pH to 7.0, in Example 7.
The respective detection levels of the microRNAs in the Examples 6 and 7 are shown in Table 1.

### Example 8 Detection of MicroRNAs from Serum Preserved after Being Mixed with Glycine Hydrochloride, Performed without Mixing Base

In Example 8, the same procedure as in Example 1 was performed except that no base was mixed. The detection level of the microRNAs in Example 8 is shown in Table 1.

### Comparative Example 1 Detection of MicroRNAs from Serum Preserved without Mixing Acid, Performed without Mixing Base

In Comparative Example 1, the same procedure as in Example 1 was performed except that no acid or base was mixed. The detection level of the microRNAs in Comparative Example 1 is shown in Table 1 and Table 2.

### Comparative Example 2 Detection of MicroRNAs from Serum Immediately After Preparation - No. 1

MicroRNAs were collected from 300 µL of serum obtained from the healthy subject X in the same manner as in the step (D) in Example 1, without performing the steps (A) to (C) in Example 1, and the collected microRNAs were detected in the same manner as in the step (E) in Example 1. The detection level of the microRNAs is shown in Table 1 and Table 2.

**[Table 1]**

| Detection Levels of MicroRNAs in Examples 1 to 8 and Comparative Examples 1 and 2 | | | | | | | |
|---|---|---|---|---|---|---|---|
| | Acid mixed | pH after | Preservat ion time | Base mixed | pH after | Total value of | Fluoresc ence |
| | | mixing with Acid | | | mixing with base | fluorescen ce intensities | intensitie s relative to before preservat ion |
| Example 1 | Glycine hydrochlo ride | 2.8 | 48 hours | Tris(hydroxymeth yl)aminomethane | 7.5 | 9.81 × 10⁵ | 115.2% |
| Example 2 | Glycine hydrochlo ride | 2.8 | 48 hours | Tris(hydroxymeth yl)aminomethane | 7.7 | 9.51 × 10⁵ | 111.7% |
| Example 3 | Glycine hydrochlo ride | 2.8 | 48 hours | Tris(hydroxymeth yl)aminomethane | 8.0 | 1.10 × 10⁶ | 128.8% |
| Example 4 | Glycine hydrochlo ride | 2.8 | 48 hours | Tris(hydroxymeth yl)aminomethane | 8.5 | 8.99 × 10⁵ | 105.6% |
| Example 5 | Glycine hydrochlo ride | 2.8 | 48 hours | Tris(hydroxymeth yl)aminomethane | 9.0 | 7.89 × 10⁵ | 92.7% |
| Example 6 | Glycine hydrochlo ride | 2.8 | 48 hours | Tris(hydroxymeth yl)aminomethane | 6.0 | 7.19 × 10⁵ | 84.5% |
| Example 7 | Glycine hydrochlo ride | 2.8 | 48 hours | Tris(hydroxymeth yl)aminomethane | 7.0 | 7.23 × 10⁵ | 84.9% |
| Example 8 | Glycine hydrochlo ride | 2.8 | 48 hours | None | 2.8 | 6.25 × 10⁵ | 73.4% |
| Compara tive Example 1 | None | 7.5 | 48 hours | None | 7.5 | 2.46 × 10⁵ | 28.9% |
| Compara tive Example 2 | None | 7.5 | 0 hours | None | 7.5 | 8.51 × 10⁵ | 100.0% |

In cases where the serum was preserved at 23°C for 48 hours after being mixed with the acid to adjust the pH to 2.8, and then the base was mixed therewith to adjust the pH within the range of from 7.5 to 8.5 (Examples 1 to 4), microRNAs were detected at a level equal to or higher (100% or more) than that in the case of using the serum immediately after the preparation (Comparative Example 2). In cases where the base was mixed to adjust the pH to 9.0 (Example 5), as well, microRNAs were detected at a level of 90% or more than that in the case of using the serum immediately after the preparation (Comparative Example 2). Further, in cases where the base was mixed to adjust the pH to 6.0 or 7.0 (Examples 6 or 7), as well, microRNAs were detected at a level equal (70% or more) to that in the case of using the serum immediately after the preparation (Comparative Example 2). In addition, also in cases where the base was not mixed (Example 8), microRNAs were detected at a level equal (70% or more) to that in the case of using the serum immediately after the preparation (Comparative Example 2).

On the other hand, in cases where the serum was preserved at 23°C for 48 hours without mixing the acid therewith (Comparative Example 1), the detection level of the microRNAs was decreased to less than 30% as compared with that in the case of using the serum immediately after the preparation (Comparative Example 2). This is thought to be the result of the microRNAs having been degraded during the preservation, due to not mixing the acid.

The above results revealed that it is important for the detection of microRNAs to preserve serum after mixing an acid therewith, and particularly that it is important for the detection of microRNAs to mix a base with the serum after the preservation to adjust the pH within the range of from 7.5 to 9.0.

### Example 9 Detection of MicroRNAs from Serum Preserved after Being Mixed with Glycine Hydrochloride - No. 4

The same procedure as in Example 1 was performed except that 45 µL of the 1 M aqueous solution of glycine hydrochloride was mixed to adjust the pH to 4.0, at the time of mixing the acid, and that 10 µL of the 4 M aqueous solution of tris(hydroxymethyl)aminomethane was mixed to adjust the pH to 7.7, at the time of mixing the base. The detection level of the microRNAs is shown in Table 2.

### Comparative Example 3 Detection of MicroRNAs from Serum Preserved without Mixing Acid

In Comparative Example 3, the same procedure as in Example 1 was performed except that no acid was mixed, and that 0.75 µL of the 4 M aqueous solution of tris(hydroxymethyl)aminomethane was mixed to adjust the pH to 7.7, at the time of mixing the base. The detection level of the microRNAs is shown in Table 2.

**[Table 2]**

| Detection Levels of MicroRNAs in Example 9 and Comparative Examples 1 to 3 | | | | | | | |
|---|---|---|---|---|---|---|---|
| | Acid mixed | pH after mixi ng with Acid | Preserva tion time | Base mixed | pH after mixi ng with base | Total value of fluoresc ence intensiti es | Fluoresc ence intensitie s relative to before preservat ion |
| Example 9 | Glycine hydrochl oride | 4.0 | 48 hours | Tris(hydroxymethyl)ami nomethane | 7.7 | 1.05 × 10⁶ | 123.4% |
| Compar ative Example 3 | None | 7.5 | 48 hours | Tris(hydroxymethyl)ami nomethane | 7.7 | 1.39 × 10⁵ | 16.3% |
| Compar ative Example 1 | None | 7.5 | 48 hours | None | 7.5 | 2.46 × 10⁵ | 28.9% |
| Compar ative Example 2 | None | 7.5 | 0 hours | None | 7.5 | 8.51 × 10⁵ | 100.0% |

In cases where the serum was preserved at 23°C for 48 hours after being mixed with the acid to adjust the pH to 4.0, and then the base was mixed therewith to adjust the pH to 7.7 (Example 9), microRNAs were detected at a level equal to or higher (90% or more) than that in the case of using the serum immediately after the preparation (Comparative Example 2).

On the other hand, in cases where the serum was preserved at 23°C for 48 hours without mixing the acid therewith, and then the base was mixed to adjust the pH to 7.7 (Comparative Example 3), and in cases where the base was not mixed (Comparative Example 1) after the preservation, the detection level of the microRNAs was decreased to less than 30% as compared with that in the case of using the serum immediately after the preparation (Comparative Example 2). This is thought to be the result of the microRNAs having been degraded during the preservation, due to not mixing the acid.

The above results revealed that it is important for the detection of microRNAs to mix an acid with serum before the preservation, and to adjust the pH to 4.0 or less.

### Examples 10 to 12 Detection of MicroRNAs from Serum Preserved after Being Mixed with Glycine Hydrochloride - No. 5

The same procedure as in Example 1 was performed except that the preservation time of the mixed liquid of the biological sample was changed to
2 hours in Example 10,
6 hours in Example 11 and
96 hours in Example 12,
and that 30 µL of the 4 M aqueous solution of tris(hydroxymethyl)aminomethane was mixed to adjust the pH to 7.7, at the time of mixing the base, in each of Examples 10 to 12. The respective detection levels of the microRNAs in Examples 10 to 12 are shown in Table 3.

**[Table 3]**

| Detection Levels of MicroRNAs in Examples 10 to 12 and Comparative Example 2 | | | | | | | |
|---|---|---|---|---|---|---|---|
| | Acid mixed | pH after mixing with Acid | Preservat ion time | Base mixed | pH after mixing with base | Total value of fluorescen ce intensities | Fluoresc ence intensitie s relative to before preservat ion |
| Example 10 | Glycine hydrochl oride | 2.8 | 2 hours | Tris(hydroxyme thyl)aminometh ane | 7.7 | 8.70 × 10⁵ | 102.1% |
| Example 11 | Glycine hydrochl oride | 2.8 | 6 hours | Tris(hydroxyme thyl)aminometh ane | 7.7 | 9.07 × 10⁵ | 106.5% |
| Example 12 | Glycine hydrochl oride | 2.8 | 96 hours | Tris(hydroxyme thyl)aminometh ane | 7.7 | 1.00 × 10⁶ | 117.8% |
| Comparati ve Example 2 | None | 7.5 | 0 hours | None | 7.5 | 8.51 × 10⁵ | 100.0% |

In cases where the serum was preserved at 23°C for 2, 6 or 96 hours after being mixed with the acid to adjust the pH to 2.8, and then the base was mixed therewith to adjust the pH to 7.7 (Examples 10, 11 or 12), microRNAs were detected at a level equal to or higher (100% or more) than that in the case of using the serum immediately after the preparation (Comparative Example 2).

The above results revealed that the use of the present method enables to detect microRNAs from serum which has been preserved at 23°C for at least 2 to 96 hours.

### Example 13 Detection of MicroRNAs from Serum Preserved after Being Mixed with Citric Acid - No. 1

The same procedure as in Example 1 was performed except that the serum obtained from a healthy subject Y was used, that 90 µL of a 1 M aqueous solution of citric acid (manufactured by Sigma-Aldrich Co. LLC.) was mixed to adjust the pH to 3.1, at the time of mixing the acid, and that 90 µL of the 4 M aqueous solution of tris(hydroxymethyl)aminomethane was mixed to adjust the pH to 7.8, at the time of mixing the base. The detection level of the microRNAs is shown in Table 4.

### Examples 14 and 15 Detection of MicroRNAs from Serum Preserved after Being Mixed with Citric Acid - No. 2

The same procedure as in Example 13 was performed except that, at the time of mixing the base,
63 µL of the 4 M aqueous solution of tris(hydroxymethyl)aminomethane was mixed to adjust the pH to 6.0, in Example 14, and
67.5 µL of the 4 M aqueous solution of tris(hydroxymethyl)aminomethane was mixed to adjust the pH to 7.0, in Example 15.
The detection levels of the microRNAs are shown in Table 4.

### Example 16 Detection of MicroRNAs from Serum Preserved after Being Mixed with Citric Acid, Performed without Mixing Base

The same procedure as in Example 13 was performed except that no base was mixed. The detection level of the microRNAs is shown in Table 4.

### Comparative Example 4 Detection of MicroRNAs from Serum Immediately After Preparation - No. 2

MicroRNAs were collected from 300 µL of serum obtained from the healthy subject Y in the same manner as in the step (D) in Example 1, without performing the steps (A) to (C) in Example 1, and the collected microRNAs were detected in the same manner as in the step (E) in Example 1. The detection level of the microRNAs is shown in Table 4.

**[Table 4]**

| Detection Levels of MicroRNAs in Examples 13 to 16 and Comparative Example 4 | | | | | | | |
|---|---|---|---|---|---|---|---|
| | Acid mixed | pH after mixing with Acid | Preservat ion time | Base mixed | pH after mixing with base | Total value of fluorescen ce intensities | Fluoresce nce intensitie s relative to before preservati on |
| Example 13 | Citric acid | 3.1 | 48 hours | Tris(hydroxyme thyl)aminometh ane | 7.8 | 1.10 × 10⁶ | 104.5% |
| Example 14 | Citric acid | 3.1 | 48 hours | Tris(hydroxyme thyl)aminometh ane | 6.0 | 8.95 × 10⁵ | 85.0% |
| Example 15 | Citric acid | 3.1 | 48 hours | Tris(hydroxyme thyl)aminometh ane | 7.0 | 9.00 × 10⁵ | 85.5% |
| Example 16 | Citric acid | 3.1 | 48 hours | None | 3.1 | 7.92 × 10⁵ | 75.2% |
| Comparati ve Example 4 | None | 7.5 | 0 hours | None | 7.5 | 1.05 × 10⁶ | 100.0% |

In cases where the serum was preserved at 23°C for 48 hours after being mixed with citric acid, and then the base was mixed therewith to adjust the pH to 7.8 (Example 13), microRNAs were detected at a level equal to or higher (100% or more) than that in the case of using the serum immediately after the preparation (Comparative Example 4). In cases where the base was mixed to adjust the pH to 6.0 or 7.0 after the preservation (Examples 14 or 15) and in cases where the base was not mixed (Example 16), as well, microRNAs were detected at a level equal (70% or more) to that in the case of using the serum immediately after the preparation (Comparative Example 4).

The above results revealed that the effect of mixing an acid with serum can be obtained regardless of the type of the acid, both in the case of using glycine hydrochloride and in the case of using citric acid.

### Example 17 Detection of MicroRNAs from Serum Preserved after Being Mixed with Glycine Hydrochloride - No. 6

The same procedure as in Example 1 was performed except that the serum obtained from a healthy subject Z was used, that 180 µL of the 1 M aqueous solution of glycine hydrochloride was mixed to adjust the pH to 2.0, at the time of mixing the acid, and that 60 µL of the 4 M aqueous solution of tris(hydroxymethyl)aminomethane was mixed to adjust the pH to 7.7, at the time of mixing the base. The detection level of the microRNAs is shown in Table 5.

### Comparative Example 5 Detection of MicroRNAs from Serum Immediately After Preparation - No. 3

MicroRNAs were collected from 300 µL of serum obtained from the healthy subject Z in the same manner as in the step (D) in Example 1, without performing the step (A) and the step (B) in Example 1, and the collected microRNAs were detected in the same manner as in the step (E) in Example 1. The detection level of the microRNAs is shown in Table 5 and Table 6.

**[Table 5]**

| Detection Levels of MicroRNAs in Example 17 and Comparative Example 5 | | | | | | | |
|---|---|---|---|---|---|---|---|
| | Acid mixed | pH after mixing with Acid | Preservat ion time | Base mixed | pH after mixing with base | Total value of fluorescen ce intensities | Fluoresc ence intensitie s relative to before preservat ion |
| Example 17 | Glycine hydrochlo ride | 2.0 | 48 hours | Tris(hydroxyme thyl)aminometh ane | 7.7 | 1.07 × 10⁶ | 115.0% |
| Compara tive Example 5 | None | 7.5 | 0 hours | None | 7.5 | 9.31 × 10⁵ | 100.0% |

In cases where the serum was preserved at 23°C for 48 hours after being mixed with the acid to adjust the pH to 2.0, and then the base was mixed therewith to adjust the pH to 7.7 (Example 17), microRNAs were detected at a level equal to or higher (100% or more) than that in the case of using the serum immediately after the preparation (Comparative Example 5).

The above results revealed that it is important for the detection of microRNAs to mix an acid with serum before the preservation, and to adjust the pH at least to 2.0 to 4.0.

### Example 18 Detection of MicroRNAs from Serum Preserved after Being Mixed with Glycine Hydrochloride - No. 7

The same procedure as in Example 1 was performed except that the serum obtained from the healthy subject Z was used, that Composition 2 prepared in Example 19 to be described later was mixed to adjust the pH to 2.8, at the time of mixing the acid, and that 30 µL of the 4 M aqueous solution of tris(hydroxymethyl)aminomethane was mixed to adjust the pH to 7.7, at the time of mixing the base. The detection level of the microRNAs is shown in Table 6.

**[Table 6]**

| Detection Levels of MicroRNAs in Example 18 and Comparative Example 5 | | | | | | | |
|---|---|---|---|---|---|---|---|
| | Acid mixed | pH after mixin g with Acid | Preserva tion time | Base mixed | pH after mixi ng with base | Total value of fluoresc ence intensiti es | Fluoresc ence intensiti es relative to before preserva tion |
| Example 18 | Glycine hydrochl oride in semi-solid state | 2.8 | 48 hours | Tris(hydroxymethyl)am inomethane | 7.7 | 1.04 × 10⁶ | 112.1% |
| Compar ative Example 5 | None | 7.5 | 0 hours | None | 7.5 | 9.31 × 10⁵ | 100.0% |

In cases where the serum was preserved at 23°C for 48 hours after being mixed with Composition 2, a homogeneous composition which is composed of a hydrochloride of a neutral amino acid, water and an alcohol and which is in a semi-solid state at 23°C, to adjust the pH to 2.8, and then the base was mixed therewith to adjust the pH to 7.7 (Example 18), microRNAs were detected at a level equal to or higher (100% or more) than that in the case of using the serum immediately after the preparation (Comparative Example 4).

The above results revealed that the effect of mixing an acid with serum before the preservation can be obtained, even in the case of using, as the acid, a homogeneous composition which is composed of a hydrochloride of a neutral amino acid, water and an alcohol and which is in a semi-solid state at 23°C.

### Example 19

### Compositions Composed of Glycine Hydrochloride, Water and Ethanol (Compositions 1 to 6)

### (1) Preparation of Compositions

To a 2 mL PP tube, 650 mg (65.0 wt%) of glycine hydrochloride (manufactured by FUJIFILM Wako Pure Chemical Industries, Ltd.), 20 mg (2.0 wt%) of ethanol (manufactured by FUJIFILM Wako Pure Chemical Industries, Ltd.) and 330 mg (33.0 wt%) of distilled water were added, so as to achieve a total amount of 1.0 g. The resulting mixture was mixed at 23°C for one minute using a vortex mixer, to obtain a suspension. The tube containing the suspension was heated at 75°C for 10 minutes, using a block incubator. After the heating, the dissolved state of glycine hydrochloride was evaluated by visual observation. As a result, no solid residue was observed in the aqueous solution, and it was confirmed that glycine hydrochloride had dissolved. The tube was taken out of the block incubator, and then left to stand at 23°C for 30 minutes, to obtain Composition 1.

Compositions 2 to 6 were each prepared in the same manner as described above, using glycine hydrochloride, ethanol and distilled water in the weight ratios shown in Table 7, so as to achieve a total amount of 1.0 g.

In the preparation process of each of Compositions 2 to 6, the fact that glycine hydrochloride had dissolved after the heating was confirmed by visual observation.

### (2) Evaluation of Shape

Composition 1 obtained in the section (1) above was deformed into a cube-like shape with a size of 0.5 cm × 0.5 cm × 1.0 cm height, on a plastic Petri dish, using a spatula, and the height was measured after allowing the composition to stand at 23°C for 10 minutes after the deformation. As a result, the height was 0.8 cm or more, and the shape after the deformation was retained. Accordingly, Composition 1 was determined to be in a semi-solid state.

The state of each of Compositions 2 to 6 was evaluated in the same manner as described above. As a result, the shape after the deformation was retained in each composition, and accordingly, Composition 1 was determined to be in a semi-solid state.

### (3) Evaluation of Solubility

The solubility of Composition 1 obtained in the section (1) above was evaluated.

One mL of distilled water was added to a 2 mL PP tube containing 0.1 g of Composition 1, and the dissolved state of the composition was evaluated by visual observation after allowing the tube to stand at 23°C for one minute. As a result, no solid residue was observed in the aqueous solution, and accordingly, it was determined that the composition quickly dissolved in water.

The solubility of each of Compositions 2 to 6 was evaluated in the same manner as described above. As a result, no solid residue was observed in the aqueous solution, and accordingly, it was determined that each composition quickly dissolved in water.

### Comparative Example 6

### Compositions Composed of Glycine Hydrochloride, Water and Ethanol (Compositions 7 to 20)

### (1) Preparation of Compositions

Compositions 7 to 20 were each prepared in the same manner as in the section (1) in Example 19, using glycine hydrochloride, ethanol and distilled water in the weight ratios shown in Table 7.

In the preparation process of each of Compositions 7 to 15, the fact that glycine hydrochloride had dissolved after the heating was confirmed by visual observation. On the other hand, in the preparation process of each of Compositions 16 to 20, solid residues were observed in the aqueous solution even after the heating. Therefore, it was determined that a part of glycine hydrochloride did not dissolve, and subsequent evaluations were not performed for each composition.

### (2) Evaluation of Shape

The state of each of Compositions 7 to 15 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 7 to 15 obtained in the section (1) was evaluated in the same manner as in the section (3) in Example 19. As a result, solid precipitation was observed in the aqueous solution, and accordingly, it was determined that each composition did not dissolve in water.

The results obtained in Example 19 and Comparative Example 6 are shown in Table 7.

**[Table 7]**

| Compositions Composed of Glycine Hydrochloride, Water and Ethanol (Compositions 1 to 20) | | | | | | | |
|---|---|---|---|---|---|---|---|
| | | Weight ratio of composition | | | Evaluation of composition | | |
| | | Glycine hydrochloride | Ethanol | Distilled water | Dissolved state after heating | State of composition after cooling | Dissolved state of composition after cooling |
| Example 19 | Composition 1 | 65.0 wt% | 2.0 wt% | 33.0 wt% | ○ | Semi-solid | ○ |
| | Composition 2 | 65.0 wt% | 4.0 wt% | 31.0 wt% | ○ | Semi-solid | ○ |
| | Composition 3 | 65.0 wt% | 6.0 wt% | 29.0 wt% | ○ | Semi-solid | ○ |
| | Composition 4 | 70.0 wt% | 2.0 wt% | 28.0 wt% | ○ | Semi-solid | ○ |
| | Composition 5 | 70.0 wt% | 4.0 wt% | 26.0 wt% | ○ | Semi-solid | ○ |
| | Composition 6 | 70.0 wt% | 6.0 wt% | 24.0 wt% | ○ | Semi-solid | ○ |
| Comparative Example 6 | Composition 7 | 60.0 wt% | 1.0 wt% | 39.0 wt% | ○ | Suspension | × |
| | Composition 8 | 60.0 wt% | 2.0 wt% | 38.0 wt% | ○ | Suspension | × |
| | Composition 9 | 60.0 wt% | 4.0 wt% | 36.0 wt% | ○ | Suspension | × |
| | Composition 10 | 60.0 wt% | 6.0 wt% | 34.0 wt% | ○ | Suspension | × |
| | Composition 11 | 60.0 wt% | 8.0 wt% | 32.0 wt% | ○ | Suspension | × |
| | Composition 12 | 65.0 wt% | 1.0 wt% | 34.0 wt% | ○ | Suspension | × |
| | Composition 13 | 65.0 wt% | 8.0 wt% | 27.0 wt% | ○ | Suspension | × |
| | Composition 14 | 70.0 wt% | 1.0 wt% | 29.0 wt% | ○ | Suspension | × |
| | Composition 15 | 70.0 wt% | 8.0 wt% | 22.0 wt% | ○ | Suspension | × |
| | Composition 16 | 75.0 wt% | 1.0 wt% | 24.0 wt% | × | - | - |
| | Composition 17 | 75.0 wt% | 2.0 wt% | 23.0 wt% | × | - | - |
| | Composition 18 | 75.0 wt% | 4.0 wt% | 21.0 wt% | × | - | - |
| | Composition 19 | 75.0 wt% | 6.0 wt% | 19.0 wt% | × | - | - |
| | Composition 20 | 75.0 wt% | 8.0 wt% | 17.0 wt% | × | - | - |

| | | | | | | | |
|---|---|---|---|---|---|---|---|
| Dissolved state of composition after heating: ○ = Dissolved. × = Not dissolved (residues observed). Dissolved state of composition after cooling: ○ = Dissolved, × = Not dissolved (precipitation observed). | | | | | | | |

Compositions 1 to 6 obtained in Example 19 were in a semi-solid state, and quickly dissolved in distilled water. On the other hand, Compositions 7 to 15 obtained in Comparative Example 11 were in a state of a suspension in which the precipitated solid of glycine hydrochloride was dispersed in the aqueous solution, and the solid did not quickly dissolve even when distilled water was further added. Further, in Compositions 16 to 20 obtained in Comparative Example 6, glycine hydrochloride did not dissolve even when heated.

The above results confirmed that it is possible to obtain the desired composition which is in a semi-solid state and which quickly dissolves in water, when the weight ratio of glycine hydrochloride in the composition is 65 wt% or more and 70 wt% or less, and the weight ratio of ethanol therein is 2 wt% or more and 6 wt% or less.

### Example 20

### Compositions Composed of Glycine Hydrochloride, Water and Methanol (Compositions 21 to 26)

### (1) Preparation of Compositions

Compositions 21 to 26 were each prepared in the same manner as in the section (1) in Example 19, except that: methanol (manufactured by FUJIFILM Wako Pure Chemical Corporation) was used instead of ethanol; the heating temperature was changed from 75°C to 60°C; and glycine hydrochloride, methanol and distilled water in the weights shown in Table 8 were used.

In the preparation process of each of Compositions 21 to 26, the fact that glycine hydrochloride had dissolved after the heating was confirmed by visual observation.

### (2) Evaluation of Shape

The state of each of Compositions 21 to 26 obtained in the section (1) was evaluated in the same manner as in the section (2) in Example 19. As a result, the shape after the deformation was retained in each composition, and accordingly, each composition was determined to be in a semi-solid state.

### (3) Evaluation of Solubility

The solubility of each of Compositions 21 to 26 obtained in the section (1) was evaluated in the same manner as in the section (3) in Example 19. As a result, solid precipitation was not observed in the aqueous solution, and accordingly, it was determined that each composition dissolved in water.

### Comparative Example 7

### Compositions Composed of Glycine Hydrochloride, Water and Methanol (Compositions 27 to 40)

### (1) Preparation of Compositions

Compositions 27 to 40 were each prepared in the same manner as in the section (1) in Example 20, except that glycine hydrochloride, methanol and distilled water in the weights shown in Table 8 were used.

In the preparation process of each of Compositions 27 to 35, the fact that glycine hydrochloride had dissolved after the heating was confirmed by visual observation. On the other hand, in the preparation process of each of Compositions 36 to 40, solid residues were observed in the aqueous solution even after the heating. Therefore, it was determined that glycine hydrochloride did not dissolve, and subsequent operations were not performed for each composition.

### (2) Evaluation of Shape

The state of each of Compositions 27 to 35 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 27 to 35 obtained in the section (1) was evaluated in the same manner as in the section (3) in Example 19. As a result, solid precipitation was observed in the aqueous solution, and accordingly, it was determined that each composition did not dissolve in water.

The results obtained in Example 20 and Comparative Example 7 are shown in Table 8.

**[Table 8]**

| Compositions Composed of Glycine Hydrochloride, Water and Methanol (Compositions 21 to 40) | | | | | | | |
|---|---|---|---|---|---|---|---|
| | | Weight ratio of composition | | | Evaluation of composition | | |
| | | Glycine hydrochloride | Methanol | Distilled water | Dissolved state after heating | State of composition after cooling | Dissolved state of composition after cooling |
| Example 20 | Composition 21 | 65.0 wt% | 2.0 wt% | 33.0 wt% | ○ | Semi-solid | ○ |
| | Composition 22 | 65.0 wt% | 4.0 wt% | 31.0 wt% | ○ | Semi-solid | ○ |
| | Composition 23 | 65.0 wt% | 6.0 wt% | 29.0 wt% | ○ | Semi-solid | ○ |
| | Composition 24 | 70.0 wt% | 2.0 wt% | 28.0 wt% | ○ | Semi-solid | ○ |
| | Composition 25 | 70.0 wt% | 4.0 wt% | 26.0 wt% | ○ | Semi-solid | ○ |
| | Composition 26 | 70.0 wt% | 6.0 wt% | 24.0 wt% | ○ | Semi-solid | ○ |
| Comparative Example 7 | Composition 27 | 60.0 wt% | 1.0 wt% | 39.0 wt% | ○ | Suspension | × |
| | Composition 28 | 60.0 wt% | 2.0 wt% | 38.0 wt% | ○ | Suspension | × |
| | Composition 29 | 60.0 wt% | 4.0 wt% | 36.0 wt% | ○ | Suspension | × |
| | Composition 30 | 60.0 wt% | 6.0 wt% | 34.0 wt% | ○ | Suspension | × |
| | Composition 31 | 60.0 wt% | 8.0 wt% | 32.0 wt% | ○ | Suspension | × |
| | Composition 32 | 65.0 wt% | 1.0 wt% | 34.0 wt% | ○ | Suspension | × |
| | Composition 33 | 65.0 wt% | 8.0 wt% | 27.0 wt% | ○ | Suspension | × |
| | Composition 34 | 70.0 wt% | 1.0 wt% | 29.0 wt% | ○ | Suspension | × |
| | Composition 35 | 70.0 wt% | 8.0 wt% | 22.0 wt% | ○ | Suspension | × |
| | Composition 36 | 75.0 wt% | 1.0 wt% | 24.0 wt% | × | - | - |
| | Composition 37 | 75.0 wt% | 2.0 wt% | 23.0 wt% | × | - | - |
| | Composition 38 | 75.0 wt% | 4.0 wt% | 21.0 wt% | × | - | - |
| | Composition 39 | 75.0 wt% | 6.0 wt% | 19.0 wt% | × | - | - |
| | Composition 40 | 75.0 wt% | 8.0 wt% | 17.0 wt% | × | - | - |

| | | | | | | | |
|---|---|---|---|---|---|---|---|
| Dissolved state of composition after heating: ○ = Dissolved. × = Not dissolved (residues observed). Dissolved state of composition after cooling: ○ = Dissolved, × = Not dissolved (precipitation observed). | | | | | | | |

Compositions 21 to 26 obtained in Example 20 were in a semi-solid state, and quickly dissolved in distilled water. On the other hand, Compositions 27 to 35 obtained in Comparative Example 7 were in a state of a suspension in which the precipitated solid of glycine hydrochloride was dispersed in the aqueous solution, and the solid did not quickly dissolve even when distilled water was further added. Further, in Compositions 36 to 40 obtained in Comparative Example 7, glycine hydrochloride did not dissolve even when heated.

The above results confirmed that it is possible to obtain the desired composition which is in a semi-solid state and which quickly dissolves in water, when the weight ratio of glycine hydrochloride in the composition is 65 wt% or more and 70 wt% or less, and the weight ratio of methanol therein is 2 wt% or more and 6 wt% or less, also in the case of using methanol as in the case of using ethanol.

### Comparative Example 8

### Preparation and Evaluation of Compositions Composed of Glycine Hydrochloride, Water and Acetone (Compositions 41 to 52)

### (1) Preparation of Compositions

Compositions 41 to 52 were each prepared in the same manner as in the section (1) in Example 19, except that: acetone (manufactured by FUJIFILM Wako Pure Chemical Corporation) was used instead of ethanol; the heating temperature was changed from 75°C to 60°C; and glycine hydrochloride, acetone and distilled water in the weights shown in Table 9 were used.

In the preparation process of each of Compositions 41 to 49, the fact that glycine hydrochloride had dissolved after the heating was confirmed by visual observation. On the other hand, in the preparation process of each of Compositions 50 to 52, solid residues were observed in the aqueous solution even after the heating. Therefore, it was determined that glycine hydrochloride did not dissolve, and subsequent operations were not performed for each composition.

### (2) Evaluation of Shape

The state of each of Compositions 41 to 49 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 41 to 49 obtained in the section (1) was evaluated in the same manner as in the section (3) in Example 19. As a result, solid precipitation was observed in the aqueous solution, and accordingly, it was determined that each composition did not dissolve in water.

The results obtained in Comparative Example 8 are shown in Table 9.

**[Table 9]**

| Compositions Composed of Glycine Hydrochloride, Water and Acetone (Compositions 41 to 52) | | | | | | | |
|---|---|---|---|---|---|---|---|
| | | Weight ratio of composition | | | Evaluation of composition | | |
| | | Glycine hydrochloride | Acetone | Distilled water | Dissolved state after heating | State of composition after cooling | Dissolved state of composition after cooling |
| Comparative Example 8 | Composition 41 | 60.0 wt% | 2.0 wt% | 38.0 wt% | ○ | Suspension | × |
| | Composition 42 | 60.0 wt% | 4.0 wt% | 36.0 wt% | ○ | Suspension | × |
| | Composition 43 | 60.0 wt% | 6.0 wt% | 34.0 wt% | ○ | Suspension | × |
| | Composition 44 | 65.0 wt% | 2.0 wt% | 33.0 wt% | ○ | Suspension | × |
| | Composition 45 | 65.0 wt% | 4.0 wt% | 31.0 wt% | ○ | Suspension | × |
| | Composition 46 | 65.0 wt% | 6.0 wt% | 29.0 wt% | ○ | Suspension | × |
| | Composition 47 | 70.0 wt% | 2.0 wt% | 28.0 wt% | ○ | Suspension | × |
| | Composition 48 | 70.0 wt% | 4.0 wt% | 26.0 wt% | ○ | Suspension | × |
| | Composition 49 | 70.0 wt% | 6.0 wt% | 24.0 wt% | ○ | Suspension | × |
| | Composition 50 | 75.0 wt% | 2.0 wt% | 23.0 wt% | × | - | - |
| | Composition 51 | 75.0 wt% | 4.0 wt% | 21.0 wt% | × | - | - |
| | Composition 52 | 75.0 wt% | 6.0 wt% | 19.0 wt% | × | - | - |

| | | | | | | | |
|---|---|---|---|---|---|---|---|
| Dissolved state of composition after heating: ○ = Dissolved. × = Not dissolved (residues observed). Dissolved state of composition after cooling: ○ = Dissolved. × = Not dissolved (precipitation observed). | | | | | | | |

When acetone was used instead of ethanol or methanol, the resulting composition was in the form of a suspension in which the solid of glycine hydrochloride was dispersed in the aqueous solution, and it was unable to obtain the desired composition according to the present invention.

### Comparative Example 9

### Compositions Composed of Glycine Hydrochloride, Water and Acetonitrile (Compositions 53 to 64)

### (1) Preparation of Compositions

Compositions 53 to 64 were each prepared in the same manner as in the section (1) in Example 19, except that acetonitrile (manufactured by FUJIFILM Wako Pure Chemical Corporation) was used instead of ethanol, and that glycine hydrochloride, acetonitrile and distilled water in the weights shown in Table 10 were used.

In the preparation process of each of Compositions 53 to 61, the fact that glycine hydrochloride had dissolved after the heating was confirmed by visual observation. On the other hand, in the preparation process of each of Compositions 62 to 64, solid residues were observed in the aqueous solution even after the heating. Therefore, it was determined that glycine hydrochloride did not dissolve, and subsequent operations were not performed for each composition.

### (2) Evaluation of Shape

The state of each of Compositions 53 to 61 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 53 to 61 obtained in the section (1) was evaluated in the same manner as in the section (3) in Example 19. As a result, solid precipitation was observed in the aqueous solution, and accordingly, it was determined that each composition did not dissolve in water.

The results obtained in Comparative Example 9 are shown in Table 10.

**[Table 10]**

| Compositions Composed of Glycine Hydrochloride, Water and Acetonitrile (Compositions 53 to 64) | | | | | | | |
|---|---|---|---|---|---|---|---|
| | | Weight ratio of composition | | | Evaluation of composition | | |
| | | Glycine hydrochloride | Acetonitrile | Distilled water | Dissolved state after heating | State of composition after cooling | Dissolved state of composition after cooling |
| Comparative Example 9 | Composition 53 | 60.0 wt% | 2.0 wt% | 38.0 wt% | ○ | Suspension | × |
| | Composition 54 | 60.0 wt% | 4.0 wt% | 36.0 wt% | ○ | Suspension | × |
| | Composition 55 | 60.0 wt% | 6.0 wt% | 34.0 wt% | ○ | Suspension | × |
| | Composition 56 | 65.0 wt% | 2.0 wt% | 33.0 wt% | ○ | Suspension | × |
| | Composition 57 | 65.0 wt% | 4.0 wt% | 31.0 wt% | ○ | Suspension | × |
| | Composition 58 | 65.0 wt% | 6.0 wt% | 29.0 wt% | ○ | Suspension | × |
| | Composition 59 | 70.0 wt% | 2.0 wt% | 28.0 wt% | ○ | Suspension | × |
| | Composition 60 | 70.0 wt% | 4.0 wt% | 26.0 wt% | ○ | Suspension | × |
| | Composition 61 | 70.0 wt% | 6.0 wt% | 24.0 wt% | ○ | Suspension | × |
| | Composition 62 | 75.0 wt% | 2.0 wt% | 23.0 wt% | × | - | - |
| | Composition 63 | 75.0 wt% | 4.0 wt% | 21.0 wt% | × | - | - |
| | Composition 64 | 75.0 wt% | 6.0 wt% | 19.0 wt% | × | - | - |

| | | | | | | | |
|---|---|---|---|---|---|---|---|
| Dissolved state of composition after heating: ○ = Dissolved. × = Not dissolved (residues observed). Dissolved state of composition after cooling: ○ = Dissolved. × = Not dissolved (precipitation observed). | | | | | | | |

When acetonitrile was used, the resulting composition was in the form of a suspension in which the solid of glycine hydrochloride was dispersed in the aqueous solution, and it was unable to obtain the desired composition according to the present invention.

### Comparative Example 10

### Compositions Composed of Glycine Hydrochloride and Water (Compositions 65 to 68)

### (1) Preparation of Compositions

Compositions 65 to 68 were each prepared in the same manner as in the section (1) in Example 19, except that ethanol was not used, and that glycine hydrochloride and distilled water in the weight ratios shown in Table 11 were used.

In the preparation process of each of Compositions 65 to 67, the fact that glycine hydrochloride had dissolved after the heating was confirmed by visual observation. On the other hand, in the preparation process of Composition 68, solid residues were observed in the aqueous solution even after the heating. Therefore, it was determined that glycine hydrochloride did not dissolve, and subsequent operations were not performed for the composition.

### (2) Evaluation of Shape

The state of each of Compositions 65 to 67 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 65 to 67 obtained in the section (1) was evaluated in the same manner as in the section (3) in Example 19. As a result, solid precipitation was observed in the aqueous solution, and accordingly, it was determined that each composition did not dissolve in water.

The results obtained in Comparative Example 10 are shown in Table 11.

**[Table 11]**

| Compositions Composed of Glycine Hydrochloride and Water (Compositions 65 to 68) | | | | | | |
|---|---|---|---|---|---|---|
| | | Weight ratio of composition | | Evaluation of composition | | |
| | | Glycine hydrochloride | Distilled water | Dissolved state after heating | State of composition after cooling | Dissolved state of composition after cooling |
| Comparative Example 10 | Composition 65 | 60.0 wt% | 40.0 wt% | ○ | Suspension | × |
| | Composition 66 | 65.0 wt% | 35.0 wt% | ○ | Suspension | × |
| | Composition 67 | 70.0 wt% | 30.0 wt% | ○ | Suspension | × |
| | Composition 68 | 75.0 wt% | 25.0 wt% | × | - | - |

| | | | | | | |
|---|---|---|---|---|---|---|
| Dissolved state of composition after heating: ○ = Dissolved. × = Not dissolved (residues observed). Dissolved state of composition after cooling: ○ = Dissolved. × = Not dissolved (precipitation observed). | | | | | | |

When glycine hydrochloride and water alone were used, the resulting composition was in the form of a suspension in which the solid of glycine hydrochloride was dispersed in the aqueous solution, and it was unable to obtain the desired composition according to the present invention.

The above-described results of Comparative Examples 8 to 10 revealed that it is necessary to use ethanol or methanol in addition to glycine hydrochloride and water, in order to obtain the desired composition according to the present invention, which is in a semi-solid state and which quickly dissolves in water.

### Comparative Example 11

### Evaluation of Solubility of Solid of Glycine Hydrochloride

One mL of distilled water was added to a 2 mL PP tube containing 0.1 g of glycine hydrochloride, and the dissolved state of glycine hydrochloride was evaluated by visual observation after allowing the tube to stand at 23°C for one minute. As a result, solid residues were observed in the aqueous solution, and accordingly, it was determined that glycine hydrochloride did not dissolve in water.

### Example 21

### pH Adjustment of Serum Using Compositions Composed of Glycine Hydrochloride, Water and Ethanol (Compositions 1 to 6)

To a 2 mL PP tube encapsulating 0.1 g of Composition 1 prepared in the section (1) in Example 19, 1 mL of serum prepared from blood collected from a healthy human individual was added, and the dissolved state of the composition was evaluated by visual observation after allowing the tube to stand at 23°C for one minute. As a result, no solid was observed in the serum, and it was determined that each composition quickly dissolved in the serum. From the upper layer of the serum in the tube, 0.1 mL of the serum was collected, and the measurement of the pH was performed using a handy pH meter (manufactured by HORIBA, Ltd.). As a result, the measured pH was 2.7, showing in a large decrease as compared with the pH (7.7) of the serum before the addition.

In the same manner as described above, 1 mL of the serum was added to a tube encapsulating 0.1 g of each of Compositions 2 to 6, and the dissolved state of each composition was evaluated by visual observation. As a result, no solid was observed in the serum, and it was determined that each of Composition 2 to 6 quickly dissolved in the serum. As a result of measuring the pH after allowing each tube to stand, the pH of the serum in the tube encapsulating Composition 2 or 3 was 2.7, and the pH of the serum in the tube encapsulating each of Compositions 4 to 6 was 2.6, each showing a large decrease as compared with the pH (7.7) of the serum before the addition.

### Comparative Example 12

### pH Adjustment of Serum Using Solid of Glycine Hydrochloride

In the same manner as in Example 21, 1 mL of the serum was added to a 2 mL PP tube encapsulating 65 mg of the solid (crystals) of glycine hydrochloride, and the dissolved state of the solid was evaluated by visual observation. As a result, the solid was observed in the serum, and it was determined that the solid of glycine hydrochloride did not quickly dissolve in the serum. As a result of measuring the pH of the serum after allowing the tube to stand, the measured pH was 4.5, and the degree of decrease in the pH was smaller as compared to the case of Example 21 in which each of Composition 1 to 6 was added.

The results of Example 21 and Comparative Example 12 are shown in Table 12.

**[Table 12]**

| pH Adjustment of Serum Using Compositions Composed of Glycine Hydrochloride, Water and Ethanol (Compositions 1 to 6) and Using Solid of Glycine Hydrochloride | | | |
|---|---|---|---|
| | Composition | Solubility | pH of serum |
| Example 21 | Composition 1 | ○ | 2.7 |
| | Composition 2 | ○ | 2.7 |
| | Composition 3 | ○ | 2.7 |
| | Composition 4 | ○ | 2.6 |
| | Composition 5 | ○ | 2.6 |
| | Composition 6 | ○ | 2.6 |
| Comparative Example 12 | Solid of glycine hydrochloride | × | 4.5 |

| | | | |
|---|---|---|---|
| Solubility of composition: ○ = Dissolved. × = Not dissolved (residues observed). | | | |

Each of Compositions (Compositions 1 to 6) composed of glycine hydrochloride, water and ethanol quickly dissolved in the serum, and reduced the pH of the serum. On the other hand, the solid of glycine hydrochloride did not quickly dissolve in the serum by merely being left to stand, resulting in a failure to sufficiently reduce the pH of the serum.

The above results revealed that the composition according to the present invention can also be used as a pH regulator.

### Example 22

### Compositions Composed of Alanine Hydrochloride, Water and Ethanol (Compositions 69 and 70)

Compositions 69 and 70 were each prepared in the same manner as in the section (1) in Example 19, except that alanine hydrochloride (manufactured by Sigma-Aldrich Co. LLC.) was used instead of glycine hydrochloride, and that alanine hydrochloride, ethanol and distilled water in the weights shown in Table 13 were used.

In the preparation process of each of Compositions 69 and 70, the fact that alanine hydrochloride had dissolved after the heating was confirmed by visual observation.

### (2) Evaluation of Shape

The state of each of Compositions 69 and 70 obtained in the section (1) was evaluated in the same manner as in the section (2) in Example 19. As a result, the shape after the deformation was retained in each composition, and accordingly, each composition was determined to be in a semi-solid state.

### (3) Evaluation of Solubility

The solubility of each of Compositions 69 and 70 obtained in the section (1) was evaluated in the same manner as in the section (3) in Example 19. As a result, solid precipitation was not observed in the aqueous solution, and accordingly, it was determined that each composition dissolved in water.

### Comparative Example 13

### Compositions Composed of Alanine Hydrochloride, Water and Ethanol (Compositions 71 to 80)

### (1) Preparation of Compositions

Compositions 71 to 80 were each prepared in the same manner as in the section (1) in Example 20, except that alanine hydrochloride, ethanol and distilled water in the weights shown in Table 13 were used.

In the preparation process of each of Compositions 71 to 77, the fact that alanine hydrochloride had dissolved after the heating was confirmed by visual observation. On the other hand, in the preparation process of each of Compositions 78 to 80, solid residues were observed in the aqueous solution even after the heating. Therefore, it was determined that alanine hydrochloride did not dissolve, and subsequent operations were not performed for each composition.

### (2) Evaluation of Shape

The state of each of Compositions 71 to 77 obtained in the section (1) was evaluated in the same manner as in the section (2) in Example 19. As a result, the shape after the deformation was not retained and a state in which the solid was dispersed in the solution was confirmed by visual observation, in each composition. Accordingly, each composition was determined to be a suspension, and not in a semi-solid state.

### (3) Evaluation of Solubility

The solubility of each of Compositions 71 to 77 obtained in the section (1) was evaluated in the same manner as in the section (3) in Example 19. As a result, solid precipitation was observed in the aqueous solution, and accordingly, it was determined that each composition did not dissolve in water.

The results obtained in Example 22 and Comparative Example 13 are shown in Table 13.

**[Table 13]**

| Compositions Composed of Alanine Hydrochloride, Water and Ethanol (Compositions 69 to 80) | | | | | | | |
|---|---|---|---|---|---|---|---|
| | | Weight ratio of composition | | | Evaluation of composition | | |
| | | Alanine hydrochloride | Ethanol | Distilled water | Dissolved state after heating | State of composition after cooling | Dissolved state of composition after cooling |
| Example 22 | Composition 69 | 65.0 wt% | 5.0 wt% | 30.0 wt% | ○ | Semi-solid | ○ |
| | Composition 70 | 70.0 wt% | 5.0 wt% | 25.0 wt% | ○ | Semi-solid | ○ |
| Comparative Example 13 | Composition 71 | 60.0 wt% | 0.0 wt% | 40.0 wt% | ○ | Suspension | × |
| | Composition 72 | 60.0 wt% | 5.0 wt% | 35.0 wt% | ○ | Suspension | × |
| | Composition 73 | 60.0 wt% | 10.0 wt% | 30.0 wt% | ○ | Suspension | × |
| | Composition 74 | 65.0 wt% | 0.0 wt% | 35.0 wt% | ○ | Suspension | × |
| | Composition 75 | 65.0 wt% | 10.0 wt% | 25.0 wt% | ○ | Suspension | × |
| | Composition 76 | 70.0 wt% | 0.0 wt% | 30.0 wt% | ○ | Suspension | × |
| | Composition 77 | 70.0 wt% | 10.0 wt% | 20.0 wt% | ○ | Suspension | × |
| | Composition 78 | 75.0 wt% | 0.0 wt% | 25.0 wt% | × | - | - |
| | Composition 79 | 75.0 wt% | 5.0 wt% | 20.0 wt% | × | - | - |
| | Composition 80 | 75.0 wt% | 10.0 wt% | 15.0 wt% | × | - | - |

| | | | | | | | |
|---|---|---|---|---|---|---|---|
| Dissolved state of composition after heating: ∘ = Dissolved. × = Not dissolved (residues observed). Dissolved state of composition after cooling: ∘ = Dissolved. × = Not dissolved (precipitation observed). | | | | | | | |

Compositions 69 and 70 obtained in Example 22 using alanine hydrochloride were in a semi-solid state, and quickly dissolved in distilled water. On the other hand, Compositions 71 to 77 obtained in Comparative Example 13 were in a state of a suspension in which the precipitated solid of alanine hydrochloride was dispersed in the aqueous solution, and the solid did not quickly dissolve even when distilled water was further added. Further, in Compositions 78 to 80 obtained in Comparative Example 13, alanine hydrochloride did not dissolve even when heated.

The above results confirmed that it is possible to obtain the desired composition which is in a semi-solid state and which quickly dissolves in water, when the weight ratio of alanine hydrochloride in the composition is 65 wt% or more and 70 wt% or less, and the weight ratio of ethanol therein is 5 wt%, also in the case of using alanine hydrochloride as in the case of using glycine hydrochloride.

### Comparative Example 14

### Compositions Composed of Glutamate Hydrochloride, Water and Ethanol (Compositions 81 to 92)

### (1) Preparation of Compositions

Compositions 81 to 92 were each prepared in the same manner as in the section (1) in Example 19, except that glutamate hydrochloride (manufactured by FUJIFILM Wako Pure Chemical Corporation) was used instead of glycine hydrochloride, and that glutamate hydrochloride, ethanol and distilled water in the weights shown in Table 14 were used.

In the preparation process of each of Compositions 81 to 86, the fact that glutamate hydrochloride had dissolved after the heating was confirmed by visual observation. On the other hand, in the preparation process of each of Compositions 87 to 92, solid residues were observed in the aqueous solution even after the heating. Therefore, it was determined that glutamate hydrochloride did not dissolve, and subsequent operations were not performed for each composition.

### (2) Evaluation of Shape

The state of each of Compositions 81 to 86 obtained in the section (1) was evaluated in the same manner as in the section (2) in Example 19. As a result, the shape after the deformation was not retained and no solid was observed by visual observation, in each composition. Accordingly, each composition was determined to be a solution in which glutamate hydrochloride was completely dissolved, and not in a semi-solid state. Since glutamate hydrochloride had dissolved, the evaluation of solubility as shown in the section (3) in Example 19 was not performed.

The results obtained in Comparative Example 14 are shown in Table 14.

**[Table 14]**

| Compositions Composed of Glutamate Hydrochloride, Water and Ethanol (Compositions 81 to 92) | | | | | | | |
|---|---|---|---|---|---|---|---|
| | | Weight ratio of composition | | | Evaluation of composition | | |
| | | Glutamate Hydrochloride | Ethanol | Distilled water | Dissolved state after heating | State of composition after cooling | Dissolved state of composition after cooling |
| Comparative Example 14 | Composition 81 | 30.0 wt% | 0.0 wt% | 70.0 wt% | ○ | Solution | - |
| | Composition 82 | 30.0 wt% | 5.0 wt% | 65.0 wt% | ○ | Solution | - |
| | Composition 83 | 30.0 wt% | 10.0 wt% | 60.0 wt% | ○ | Solution | - |
| | Composition 84 | 40.0 wt% | 0.0 wt% | 60.0 wt% | ○ | Solution | - |
| | Composition 85 | 40.0 wt% | 5.0 wt% | 55.0 wt% | ○ | Solution | - |
| | Composition 86 | 40.0 wt% | 10.0 wt% | 50.0 wt% | ○ | Solution | - |
| | Composition 87 | 50.0 wt% | 0.0 wt% | 50.0 wt% | × | - | - |
| | Composition 88 | 50.0 wt% | 5.0 wt% | 45.0 wt% | × | - | - |
| | Composition 89 | 50.0 wt% | 10.0 wt% | 40.0 wt% | × | - | - |
| | Composition 90 | 60.0 wt% | 0.0 wt% | 40.0 wt% | × | - | - |
| | Composition 91 | 60.0 wt% | 5.0 wt% | 35.0 wt% | × | - | - |
| | Composition 92 | 60.0 wt% | 10.0 wt% | 30.0 wt% | × | - | - |

| | | | | | | | |
|---|---|---|---|---|---|---|---|
| Dissolved state of composition after heating: ∘ = Dissolved. × = Not dissolved (residues observed). Dissolved state of composition after cooling: ∘ = Dissolved. × = Not dissolved (precipitation observed). | | | | | | | |

When glutamate hydrochloride was used, the resulting composition was in the form of a solution in which glutamate hydrochloride was completely dissolved, and it was unable to obtain the desired composition according to the present invention.

### Comparative Example 15

### Compositions Composed of Lysine Monohydrochloride, Water and Ethanol (Compositions 93 to 104)

### (1) Preparation of Compositions

Compositions 93 to 104 were each prepared in the same manner as in the section (1) in Example 19, except that lysine monohydrochloride (manufactured by FUJIFILM Wako Pure Chemical Corporation) was used instead of glycine hydrochloride, and that lysine monohydrochloride, ethanol and distilled water in the weights shown in Table 15 were used.

In the preparation process of each of Compositions 93 to 98, the fact that lysine monohydrochloride had dissolved after the heating was confirmed by visual observation. On the other hand, in the preparation process of each of Compositions 99 to 104, solid residues were observed in the aqueous solution even after the heating. Therefore, it was determined that lysine monohydrochloride did not dissolve, and subsequent operations were not performed for each composition.

### (2) Evaluation of Shape

The state of each of Compositions 93 to 98 obtained in the section (1) was evaluated in the same manner as in the section (2) in Example 19. As a result, the shape after the deformation was not retained and no solid was observed by visual observation, in each composition. Accordingly, each composition was determined to be a solution in which lysine monohydrochloride was completely dissolved, and not in a semi-solid state. Since lysine monohydrochloride had dissolved, the evaluation of solubility as shown in the section (3) in Example 19 was not performed.

The results obtained in Comparative Example 15 are shown in Table 15.

**[Table 15]**

| Compositions Composed of Lysine Monohvdrochloride, Water and Ethanol (Compositions 93 to 104) | | | | | | | |
|---|---|---|---|---|---|---|---|
| | | Weight ratio of composition | | | Evaluation of composition | | |
| | | Lysine monohydrochloride | Ethanol | Distilled water | Dissolved state after heating | State of composition after cooling | Dissolved state of composition after cooling |
| Comparative Example 15 | Composition 93 | 40.0 wt% | 0.0 wt% | 60.0 wt% | ○ | Solution | - |
| | Composition 94 | 40.0 wt% | 5.0 wt% | 55.0 wt% | ○ | Solution | - |
| | Composition 95 | 40.0 wt% | 10.0 wt% | 50.0 wt% | ○ | Solution | - |
| | Composition 96 | 50.0 wt% | 0.0 wt% | 50.0 wt% | ○ | Solution | - |
| | Composition 97 | 50.0 wt% | 5.0 wt% | 45.0 wt% | ○ | Solution | - |
| | Composition 98 | 50.0 wt% | 10.0 wt% | 40.0 wt% | ○ | Solution | - |
| | Composition 99 | 60.0 wt% | 0.0 wt% | 40.0 wt% | × | - | - |
| | Composition 100 | 60.0 wt% | 5.0 wt% | 35.0 wt% | × | - | - |
| | Composition 101 | 60.0 wt% | 10.0 wt% | 30.0 wt% | × | - | - |
| | Composition 102 | 70.0 wt% | 0.0 wt% | 30.0 wt% | × | - | - |
| | Composition 103 | 70.0 wt% | 5.0 wt% | 25.0 wt% | × | - | - |
| | Composition 104 | 70.0 wt% | 10.0 wt% | 20.0 wt% | × | - | - |

| | | | | | | | |
|---|---|---|---|---|---|---|---|
| Dissolved state of composition after heating: ∘ = Dissolved. × = Not dissolved (residues observed). Dissolved state of composition after cooling: ∘ = Dissolved. × = Not dissolved (precipitation observed). | | | | | | | |

When lysine monohydrochloride was used, the resulting composition was in the form of a solution in which lysine monohydrochloride was completely dissolved, and it was unable to obtain the desired composition according to the present invention.

### Comparative Example 16

### Compositions Composed of Lysine Dihydrochloride, Water and Ethanol (Compositions 105 to 116)

### (1) Preparation of Compositions

Compositions 105 to 116 were each prepared in the same manner as in the section (1) in Example 19, except that lysine dihydrochloride (manufactured by FUJIFILM Wako Pure Chemical Corporation) was used instead of glycine hydrochloride, and that lysine dihydrochloride, ethanol and distilled water in the weights shown in Table 16 were used.

In the preparation process of each of Compositions 105 to 113, the fact that lysine dihydrochloride had dissolved after the heating was confirmed by visual observation. On the other hand, in the preparation process of each of Compositions 114 to 116, solid residues were observed in the aqueous solution even after the heating. Therefore, it was determined that lysine dihydrochloride did not dissolve, and subsequent operations were not performed for each composition.

### (2) Evaluation of Shape

The state of each of Compositions 105 to 113 obtained in the section (1) was evaluated in the same manner as in the section (2) in Example 19. As a result, the shape after the deformation was not retained and no solid was observed by visual observation, in each composition. Accordingly, each composition was determined to be a solution in which lysine dihydrochloride was completely dissolved, and not in a semi-solid state. Since lysine dihydrochloride had dissolved, the evaluation of solubility as shown in the section (3) in Example 19 was not performed.

The results obtained in Comparative Example 16 are shown in Table 16.

**[Table 16]**

| Compositions Composed of Lysine Dihydrochloride, Water and Ethanol (Compositions 105 to 116) | | | | | | | |
|---|---|---|---|---|---|---|---|
| | | Weight ratio of composition | | | Evaluation of composition | | |
| | | Lysine dihydrochloride | Ethanol | Distilled water | Dissolved state after heating | State of composition after cooling | Dissolved state of composition after cooling |
| Comparative Example 16 | Composition 105 | 40.0 wt% | 0.0 wt% | 60.0 wt% | ○ | Solution | - |
| | Composition 106 | 40.0 wt% | 5.0 wt% | 55.0 wt% | ○ | Solution | - |
| | Composition 107 | 40.0 wt% | 10.0 wt% | 50.0 wt% | ○ | Solution | - |
| | Composition 108 | 50.0 wt% | 0.0 wt% | 50.0 wt% | ○ | Solution | - |
| | Composition 109 | 50.0 wt% | 5.0 wt% | 45.0 wt% | ○ | Solution | - |
| | Composition 110 | 50.0 wt% | 10.0 wt% | 40.0 wt% | ○ | Solution | - |
| | Composition 111 | 60.0 wt% | 0.0 wt% | 40.0 wt% | ○ | Solution | - |
| | Composition 112 | 60.0 wt% | 5.0 wt% | 35.0 wt% | ○ | Solution | - |
| | Composition 113 | 60.0 wt% | 10.0 wt% | 30.0 wt% | ○ | Solution | - |
| | Composition 114 | 70.0 wt% | 0.0 wt% | 30.0 wt% | × | - | - |
| | Composition 115 | 70.0 wt% | 5.0 wt% | 25.0 wt% | × | - | - |
| | Composition 116 | 70.0 wt% | 10.0 wt% | 20.0 wt% | × | - | - |

| | | | | | | | |
|---|---|---|---|---|---|---|---|
| Dissolved state of composition after heating: ∘ = Dissolved. × = Not dissolved (residues observed). Dissolved state of composition after cooling: ∘ = Dissolved. × = Not dissolved (precipitation observed). | | | | | | | |

When lysine dihydrochloride was used, the resulting composition was in the form of a solution in which lysine dihydrochloride was completely dissolved, and it was unable to obtain the desired composition according to the present invention.

## Claims

1. A method of detecting a microRNA in a biological sample, the detection method comprising the steps of:
(A) mixing an acid with said biological sample to obtain a mixed liquid of said biological sample;
(B) preserving said mixed liquid of said biological sample obtained in the step (A);
(D) collecting said microRNA from said mixed liquid of said biological sample obtained in the step (B); and
(E) detecting said microRNA collected in the step (D).

2. The detection method of claim 1, wherein said mixed liquid of said biological sample obtained in said step (A) has a pH of from 2.0 to 4.0.

3. The detection method of claim 1 or 2, comprising, before said step (D), the step (C) of mixing a base with said mixed liquid of said biological sample obtained in said step (B), to adjust the pH of said mixed liquid within the range of from 6.0 to 9.0.

4. The detection method of claim 3, wherein said pH of said mixed liquid is adjusted within the range of from 7.5 to 9.0 in said step (C).

5. The detection method of any one of claims 1 to 4, wherein said biological sample is blood, serum or plasma.

6. The detection method of any one of claims 1 to 5, wherein said acid used in said step (A) is glycine hydrochloride, alanine hydrochloride, citric acid, hydrochloric acid, sulfuric acid, acetic acid, lactic acid or oxalic acid.

7. The detection method of any one of claims 1 to 5, wherein said acid used in said step (A) is a homogeneous composition which is composed of a hydrochloride of a neutral amino acid, water and an alcohol, and which is in a semi-solid state at 23°C.

8. The detection method of any one of claims 1 to 7, wherein said mixed liquid of said biological sample is preserved for 96 hours or less in said step (B).

9. The detection method of any one of claims 1 to 8, wherein said mixed liquid of said biological sample is preserved at a temperature of from 1 to 30°C in said step (B).

10. The detection method of claim 3 or 4, wherein said base used in said step (C) is tris(hydroxymethyl)aminomethane, N-[tris(hydroxymethyl)methyl]glycine, triethanolamine, sodium hydroxide, potassium hydroxide, calcium hydroxide, N,N-bis(2-hydroxyethyl)glycine, N-(2-hydroxyethyl)piperazine-N'-2-ethanesulfonic acid or 2-hydroxy-3-[tris(hydroxymethyl)methylamino]-1-propanesulfonic acid.

11. The detection method of any one of claims 1 to 10, wherein said microRNA is detected by the microarray analysis in said step (E).

12. The detection method of any one of claims 1 to 11, wherein said microRNA is a microRNA to be used as a disease marker.

13. The detection method of claim 12, wherein said disease is cancer or dementia.

14. A composition which is used as said acid in said step (A) in the detection method of claim 1, wherein said composition is a homogeneous composition which is composed of a hydrochloride of a neutral amino acid, water and an alcohol, and which is in a semi-solid state at 23°C.

15. The composition of claim 14, wherein the weight ratio of said hydrochloride of a neutral amino acid in said composition is 65 wt% or more and 70 wt% or less, and the weight ratio of said alcohol therein is 2 wt% or more and 6 wt% or less.

16. The composition of claim 14 or 15, wherein said hydrochloride of a neutral amino acid is glycine hydrochloride or alanine hydrochloride.

17. The composition of any one of claims 14 to 16, wherein said alcohol is methanol or ethanol.

18. A container for preserving a biological sample, wherein the composition of any one of claims 14 to 17 is encapsulated in said container.
